# EUROPEAN PATENT SPECIFICATION

(11) **EP 2 035 437 B1**
(45) Date of publication and mention of the grant of the patent: **18.08.2010**
(21) Application number: 07765528.0
(22) Date of filing: 26.06.2007
(51) Int. Cl.: C07F 5/02, C07C 29/143, C07C 29/147, C07C 209/44, C07C 209/50, C07D 307/06, C01B 35/00

(54) **BORANE ETHER COMPLEXES**
BORANETHERKOMPLEXE
COMPLEXES BORANE-ÉTHER

(30) Priority: 26.06.2006 US 816557 P
(43) Date of publication of application: 18.03.2009
(73) Proprietor: BASF SE, 67056 Ludwigshafen (DE)
(72) Inventor: BURKHARDT, Elizabeth, Bridgeville, PA 15017 (US); ATTLESEY, Alex J., Cranberry Township, PA 16066 (US)
(86) International application number: PCT/EP2007/056171
(87) International publication number: WO 2008/000678

(56) References cited:
- COREY E J ET AL: "Reduction of Carbonyl Compounds with Chiral Oxazaborolidine Catalysts: A New Paradigm for Enantioslsctive Catalysis and a powerful New synthetic Method" ANGEWANDTE CHEMIE, INTERNATIONAL EDITION, vol. 37, no. 15, 17 August 1998 (1998-08-17), pages 1986-2012, XP002427347 ISSN: 1433-7851 cited in the application
- HUTTENHAIN S H: "Asymmetric induction from ethyl lactate in the reduction of acetophenone to phenylethanol" SYNTHETIC COMMUNICATIONS, vol. 36, no. 2, 1 February 2006 (2006-02-01), pages 175-180, XP009090593 ISSN: 0039-7911 1532-2432
- BROWN H C ET AL: "Molecular Addition Compounds. 11. N-Ethyl-N-isopropylaniline-Borane, A Superior Reagent for Hydroborations and Reductions" JOURNAL OF ORGANIC CHEMISTRY, vol. 63, no. 15, 1998, pages 5154-5163, XP009090580 ISSN: 0022-3263
- BROWN H C ET AL: "Molecular addition compounds. 15. Synthesis, hydroboration, and reduction studies of new, highly reactive tert-butyldialkylamine-bor ane adducts" JOURNAL OF ORGANIC CHEMISTRY, vol. 64, no. 17, 20 August 1999 (1999-08-20), pages 6263-6274, XP009090574 ISSN: 0022-3263

## Description

### Field of the Invention

The present invention relates to new borane complexes with substituted tetrahydrofuran ethers and a method of using new borane complexes with substituted tetrahydrofuran ethers for organic reactions.

### Background of the Invention

Diborane (B₂H₆) is a toxic and pyrophoric gas that is very readily hydrolysed and oxidised. It must be handled with utmost precautions and must be shipped and stored at temperatures below -20°C. In order to reduce the hazards of diborane, complexes of borane (BH₃) with donor molecules like tetrahydrofuran, sulfides, amines and phosphines are invariably used for organic reactions, especially for the reduction of functional groups and for hydroboration reactions with alkenes and alkynes. Functional groups reduced by such borane complexes include aldehyde, ketone, lactone, epoxide, ester, amide, oxime, imine and nitrile groups.

The most used source of borane is a tetrahydrofuran (THF) solution of the borane-THF complex, which is commercially available, usually with a concentration of 1 mol/l. However, the borane-THF complex is prone to thermal decomposition by ether cleavage of the tetrahydrofuran ring, leading to butoxyboranes and ultimately to tributylborate as decomposition products. According to US 6,048,985, the storage stability of borane-THF complex in THF solution is increased significantly at low temperatures, even for solutions with higher concentrations.

It is well known that tetrahydrofuran forms a stronger complex with borane than linear ethers like diethyl ether or the series of glymes derived from ethylene glycol. Other ethers with five-membered ring structures have not been examined so far. Borane reagents with other complexing agents are available but suffer from inherent disadvantages. For example, sulfide boranes are highly concentrated but their commercial use is limited because of their strong odor. The reactivity of amine boranes is frequently not sufficient to reduce a specific functional group. Moreover, such complexing agents are sometimes difficult to remove from the reaction mixture and isolation of the desired product may become laborious.

Depending on the substrate, hydroboration and reduction reactions using borane reagents can often be accomplished at ambient temperature or lower temperature to increase selectivity. However, borane reagents are sometimes employed by heating the borane reagent together with a substrate (i.e., a compound to be reacted with the borane reagent) in a reaction vessel and preventing the escape of evolved gaseous diborane from the reaction vessel. Due to the low thermal stability of some borane reagents and to the possible loss of gaseous diborane, usually an excess of the borane reagent is used in such transformations. When the reaction is finished the reaction mixture is typically quenched, e.g. with an alcohol, to destroy any remaining borane reagent before work-up. It is evident that the nature of the complexing agent strongly affects the stability and reactivity of the borane reagent as well as pressure and temperature at which a reaction can be run and the work-up procedure.

Therefore, it is desirable to develop new borane reagents with improved stability and reactivity properties and methods of using them in order to achieve a better efficiency for organic transformations employing borane reagents.

### Summary of the Invention

The present invention provides new borane ether complexes comprising substituted tetrahydrofurans as the complexing agent and solvent. Another object of the present invention was the development of methods of using the new borane ether complexes for organic reactions.

Accordingly, new borane ether complexes of the formula 1 have been found, 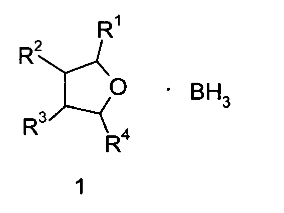 wherein
R¹ to R⁴ represent independently from each other hydrogen, C₁ - C₁₀-alkyl, C₃ - C₆-cycloalkyl, phenyl, benzyl, substituted phenyl or a substituent of the formula CH₂OR⁵ , wherein R⁵ is C₁ - C₁₀-alkyl, C₃ - C₆-cycloalkyl, or -[-CHR⁶CH₂O-]ₙ-R⁷, wherein R⁶ is hydrogen or methyl, R⁷ is C₁ - C₁₀-alkyl and n is an integer between 1 and 20,
or two adjacent substituents R¹ to R⁴ together are a divalent group selected from the group consisting of -CH₂CH₂-, -CH(CH₃)CH₂-, -CH₂CH₂CH₂-, -CH(CH₃)CH(CH₃)-, -CH(CH₂CH₃)CH₂-, -C(CH₃)₂C(CH₃)₂-, -CH₂C(CH₃)₂CH₂- and -(CH₂)₆- to form with the -CH-CH- moiety of the tetrahydrofuran ring a cyclic structure,
with the provision that at least one of the substituents R¹ to R⁴ is not hydrogen.

The new borane ether complexes of the present invention can be prepared by similar methods used for the synthesis of the borane-tetrahydrofuran complex. One method comprises the in situ generation of borane from sodium borohydride and boron trifluoride in the respective substituted tetrahydrofuran (c.f. A. Pelter, K. Smith, H. C. Brown, "Borane Reagents", pp. 421 - 422, Academic Press 1988). Preferably, the new borane ether complexes are made in high purity by direct addition of gaseous diborane to the respective substituted tetrahydrofuran.

The new borane ether complexes of the present invention can be employed for a large number of organic transformations. Examples are the reduction of functional groups and hydroboration reactions with alkenes and alkynes. Functional groups reduced by such borane complexes may for example include aldehyde, ketone, lactone, epoxide, ester, amide, oxime, imine, carboxylic acid and nitrile groups.

The new borane ether complexes of the present invention offer numerous advantages compared to the known borane complex of unsubstituted tetrahydrofuran. Due to the generally higher boiling point (e.g. 78°C for 2-methyltetrahydrofuran versus 66°C for THF) and flash point (e.g. -11°C for 2-methyltetrahydrofuran versus -17°C for THF) of the substituted tetrahydrofurans compared to unsubstituted tetrahydrofuran the compounds pose lower flammability hazards. Depending on the nature, number and position of the substituents attached to the five-membered ring of the new compounds 1, the new borane ether complexes are less polar and the ethereal complexing agent shows a reduced miscibility with water compared to unsubstituted tetrahydrofuran, which facilitates work-up procedures for the reaction mixtures. Moreover, the energy released upon thermal decomposition of the new compounds is in most cases much lower than for borane-tetrahydrofuran, which results in an important safety advantage of the new compounds.

When employed in enantioselective reductions of ketones with methyl-substituted chiral oxazaborolidine catalysts (known as MeCBS catalysts, c.f. Corey, E.J. et al., Angew. Chem. Int. Ed., 37, 1986-2012 (1998)), it was surprisingly found that the enantiomeric excess obtained with the new borane ether complexes is higher than with borane-tetrahyd rofu ran.

### Brief description of the drawings

Figure 1 illustrates shelf-life or decomposition studies of 0.88M solutions of borane-2-methyltetrahydrofuran in 2-methyltetrahydrofuran (prepared according to example 1) at ambient temperature with and without addition of sodium borohydride.
Figure 2 illustrates shelf-life or decomposition studies of 0.88M solutions of borane-2-methyltetrahydrofuran in 2-methyltetrahydrofuran (prepared according to example 1) at ambient temperature and at 0-5°C.
Figure 3 illustrates shelf-life or decomposition studies of 0.88M solutions of borane-2-methyltetrahydrofuran in 2-methyltetrahydrofuran (prepared according to example 1) with addition of sodium borohydride at ambient temperature and at 0-5°C.
Figure 4 illustrates shelf-life or decomposition studies of 0.88M solutions of borane-2-methyltetrahydrofuran in 2-methyltetrahydrofuran (prepared according to example 1) at 0-5°C with and without addition of sodium borohydride.
Figure 5 compares shelf-life or decomposition studies of 1 M solutions of borane-2-methyltetrahydrofuran in 2-methyltetrahydrofuran and borane-tetrahydrofuran in tetrahydrofuran at ambient temperature with and without addition of sodium borohydride.
Figure 6 illustrates the decomposition of borane-2,5-dimethyltetrahydrofuran in 2,5-dimethyltetrahydrofuran at ambient temperature.

### Detailed Description of the Invention

The new borane ether complexes of the present invention have chemical structures according to the general formula 1, 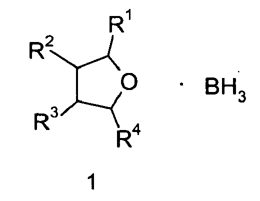 wherein
R¹ to R⁴ represent independently from each other hydrogen, C₁ - C₁₀-alkyl, C₃ - C₆-cycloalkyl, phenyl, benzyl, substituted phenyl or a substituent of the formula CH₂OR⁵ , wherein R⁵ is C₁ - C₁₀-alkyl, C₃ - C₆-cycloalkyl or -[-CHR⁶CH₂O-]ₙ-R⁷, wherein R⁶ is hydrogen or methyl, R⁷ is C₁ - C₁₀-alkyl and n is an integer between 1 and 20,
or two adjacent substituents R¹ to R⁴ together are a divalent group selected from the group consisting of -CH₂CH₂-, -CH(CH₃)CH₂-, -CH₂CH₂CH₂-, -CH(CH₃)CH(CH₃)-, -CH(CH₂CH₃)CH₂-, -C(CH₃)₂C(CH₃)₂-, -CH₂C(CH₃)₂CH₂- and -(CH₂)₆- to form with the -CH-CH- moiety of the tetrahydrofuran ring a cyclic structure,
with the provision that at least one of the substituents R¹ to R⁴ is not hydrogen.

As used herein, the term "C₁ - C₁₀-alkyl" denotes a branched or an unbranched saturated hydrocarbon group comprising between 1 and 4 carbon atoms. Examples are methyl, ethyl, propyl, isopropyl, n-butyl, isobutyl, sec-butyl, t-butyl, hexyl and octyl.

The term "C₃ - C₆-cycloalkyl " denotes a saturated hydrocarbon group comprising between 3 and 6 carbon atoms including a mono- or polycyclic structural moiety. Examples are cyclopropyl, cyclobutyl, cyclopentyl and cyclohexyl.

The term "substituted phenyl" denotes a phenyl group with at least one hydrogen atom replaced by a halide atom like fluorine, chlorine, bromine or iodine or by an C₁ - C₈-alkoxy group.

The term "C₁ - C₈-alkoxy" denotes a group derived from a branched or an unbranched aliphatic monoalcohol comprising between 1 and 8 carbon atoms. Examples are methoxy, ethoxy, propoxy, isopropoxy, n-butoxy, isobutoxy and n-pentoxy.

The term "adjacent" denotes the relative position of two groups that are separated by three bonds.

It should be emphasized that all stereoisomers are included in cases where more than one substituent R¹ to R⁴ present at the five-membered ring in compounds according to formula 1 is not hydrogen.

The new borane ether complexes of the present invention can be prepared by the reaction of diborane with the respective substituted tetrahydrofuran. In order to allow for this reaction the diborane can be brought in contact with the respective substituted tetrahydrofuran by any method, including its in situ formation, e.g. from alkali metal borohydrides. The new borane ether complexes of the present invention are preferably prepared in high purity by direct addition of gaseous diborane to the respective substituted tetrahydrofuran. In this synthesis the substituted tetrahydrofuran is usually present in large excess compared to the diborane and, therefore, serves both as complexing agent for the borane and as solvent for the newly formed borane ether complex. Of course, other solvents with poorer complexing ability to borane, that are at least partially miscible with the respective substituted tetrahydrofuran, may also be present, for example linear ethers like diethyl ether or hydrocarbons, like pentane, hexane, heptane, cyclohexane, toluene or xylenes.

The concentration of the new borane ether complexes in the respective substituted tetrahydrofuran containing solvent or solvent mixture is generally in the range between 0.01 and 3 mol/l, preferably between 0.1 and 1.5 mol/l, most preferably between 0.5 and 1.25 mol/l.

The formation reaction for the new borane ether complexes of the present invention is usually exothermic. Owing to the thermal instability of the borane ether complexes in general it is advisable to control the temperature of the reaction mixture in course of the reaction. In order to avoid side reactions and formation of impurities the temperature of the reaction mixture should be below ambient temperature, preferably below 0°C and most preferably below -30°C.

The way in which the gaseous diborane is brought into contact with the ethereal solution is therefore of significant importance to controlling the exothermic reaction of borane complex formation. If a dip tube or a nozzle submerged under the surface of the ethereal solution is used to add gaseous diborane to the solution, intensive cooling together with vigorous stirring and a slow addition rate is recommended to prevent localized heating. The same is true when diborane is added to the headspace of a reaction vessel containing the required ethereal solution, although in this case the reaction will take place in the gas phase and over the whole surface of the liquid phase. If necessary, the diborane might be diluted with an inert gas like nitrogen or argon before it is brought into contact with the ethereal complexing agent. Preparation of ethereal borane reagents from cryogenically stored diborane yields higher purity borane reagents than when produced by in situ routes. Moreover, preparation of ethereal borane reagents from sodium borohydride leads to sodium borohydride and sodium tetrafluoroborate impurities that can be detrimental to asymmetric reductions.

It is well known that etheral borane complexes tend to undergo thermal decompositions. For the known borane-tetrahydrofuran complex thermal decomposition occurs by ether cleavage of the tetrahydrofuran ring. Borane-tetrahydrofuran complex can thermally decompose during the course of reactions or if stored improperly. Storage temperatures above 5°C lead to appreciable decomposition in a matter of weeks. During storage the primary mode of decomposition is by tetrahydrofuran ring-opening (ether cleavage). The first intermediate, monobutoxyborane, is never observed in ¹¹B NMR spectra of partially degraded solutions of the borane-tetrahydrofuran complex. Obviously, it readily disproportionates giving borane-tetrahydrofuran and dibutoxyborane (DiMare, M., J. Org. Chem. 1996, 61(24), 8378-8385). Dibutoxyborane only slowly disproportionates or further reacts and is observed in the ¹¹B NMR spectrum at δ = 27 ppm as a doublet (¹J (¹¹B¹H) = 159 Hz). Tributylborate is ultimately the end product after all three borane (B-H) bonds have reacted.

The storage stability of solutions of the known borane-tetrahydrofuran complex at different concentrations can be increased by keeping the solutions at low temperatures (c.f. US 6,048,985) and/or by addition of small amounts (usually less than 1 mol/l, preferably between 0.001 and 0.02 mol/l) of hydride sources like sodium borohydride, potassium borohydride or alkali metal hydrides such as lithium hydride, sodium hydride or potassium hydride (c.f. US 3,634,277). From our ¹¹B NMR spectroscopic studies it appears that hydride addition to solutions of borane-tetrahydrofuran complex gives rise to the formation of the B₃H₈-anion, which may act as the actual stabilizing agent.

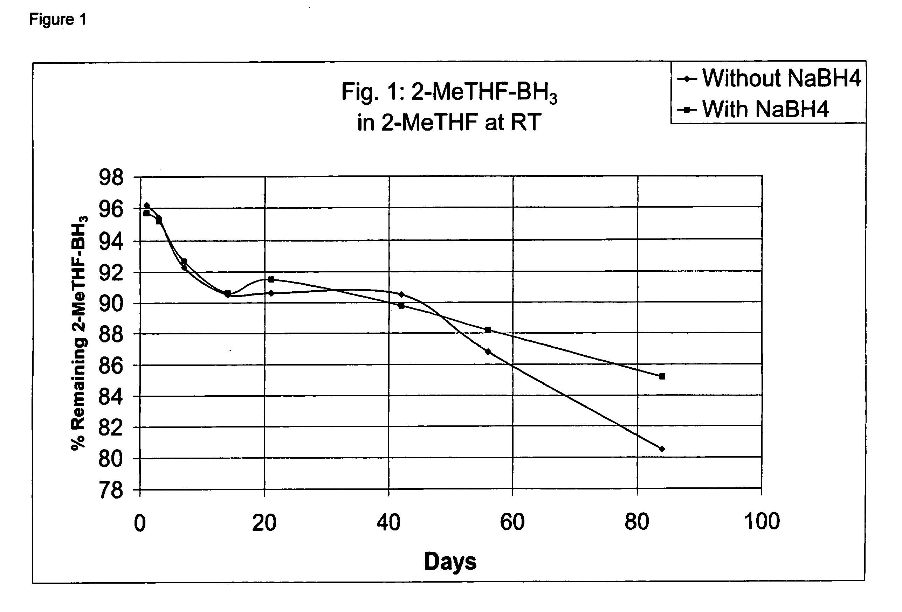

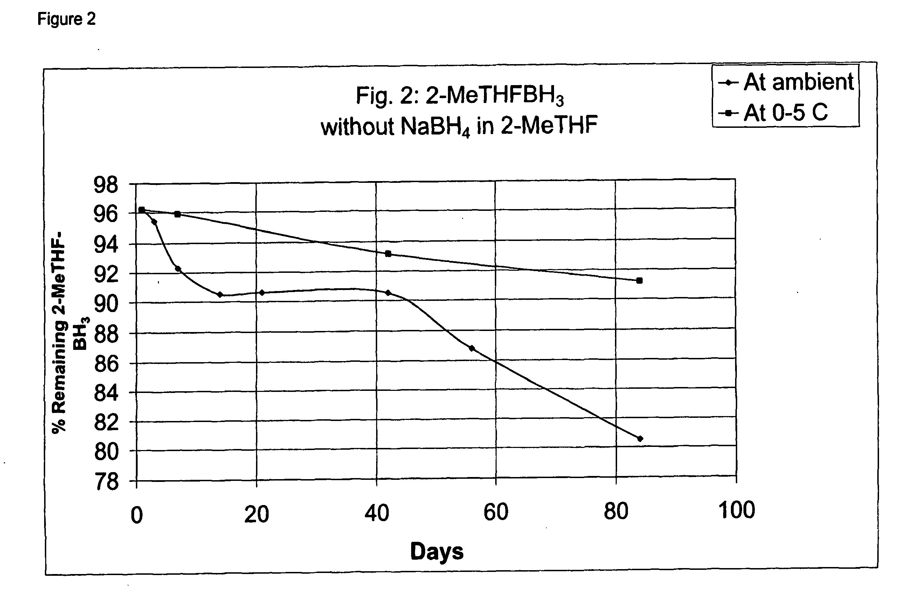

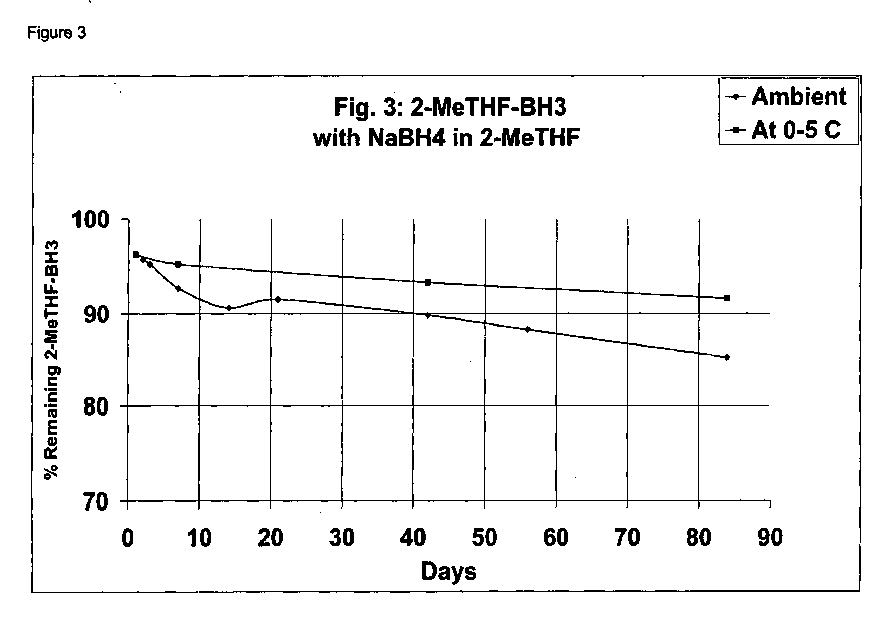

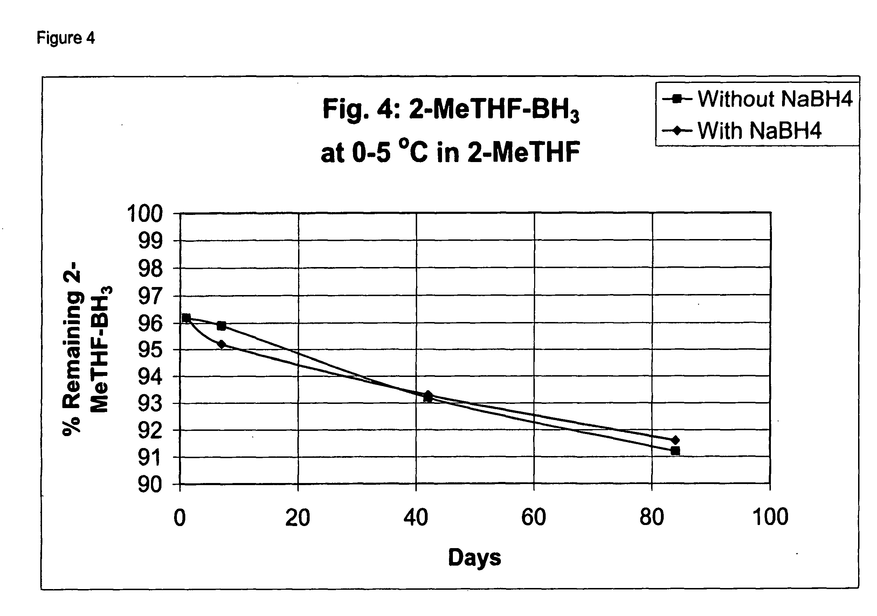

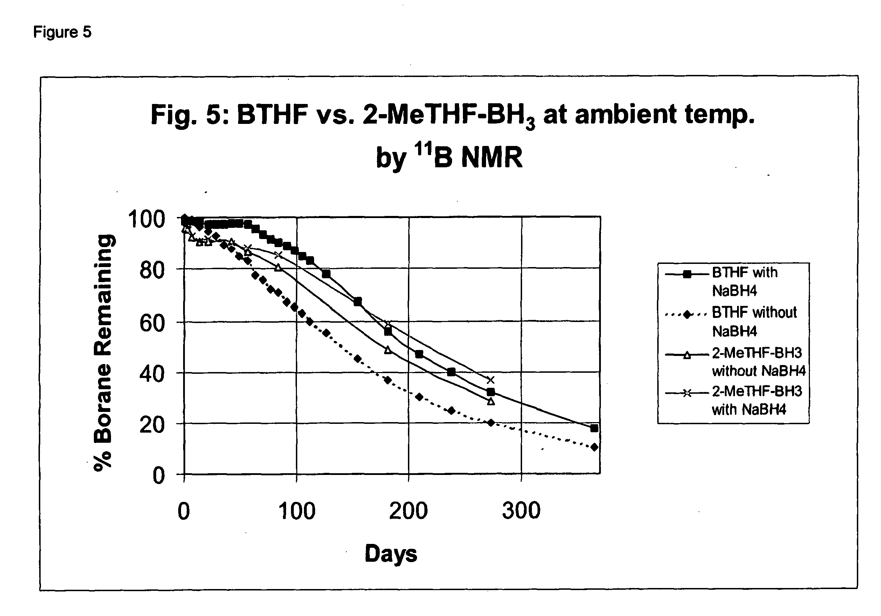

Therefore, shelf-life studies have been performed with solutions of the new borane ether complexes of the present invention under various conditions. For example, Figures 1 to 4 show the results of shelf-life or decomposition studies of 0.88M solutions of borane-2-methyltetrahydrofuran in 2-methyltetrahydrofuran at different temperatures (ambient or 0-5°C) with or without addition of sodium borohydride. The increase in storage stability is more pronounced when lowering the temperature than by addition of sodium borohydride. Figure 5 compares shelf-life or decomposition studies of 1 M solutions of borane-2-methyltetrahydrofuran in 2-methyltetrahydrofuran and borane-tetrahydrofuran in tetrahydrofuran at ambient temperature with and without addition of sodium borohydride. From this data it can be seen that the borane-2-methyltetrahydrofuran complex in 2-methyltetrahydrofuran is slightly more stable than the commercially available borane-tetrahydrofuran complex in tetrahydrofuran.

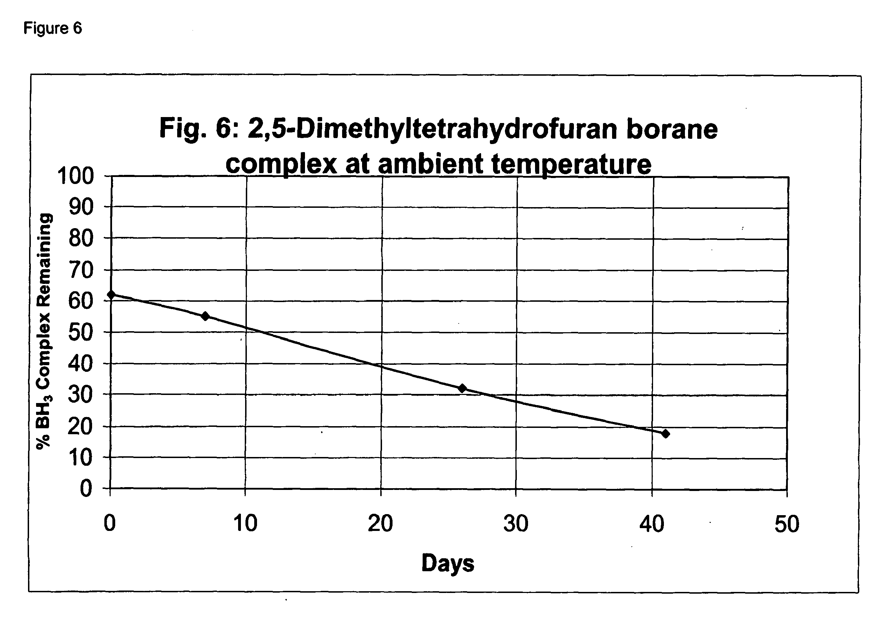

The borane complex of 2,5-dimethyltetrahydrofuran is less stable and decomposes faster at ambient temperature with a rate of about 1 % per day, see Figure 6.

Owing to the high energy content of boranes, the energy release in thermal degradation of some of the new borane ether complexes of the present invention has been studied by Differential Scanning Calorimetry (DSC) and compared with the data for the commercially available borane-tetrahydrofuran complex in tetrahydrofuran. The results are summarized in Table 1:

**Table 1: Energy release for different borane derivatives**

| Compound* | Onset-temperature (°C) | ΔH (J/g) |
|---|---|---|
| BH₃ - THF (1M) | 134 | -250 |
| BH₃ - THF (1M) with 0.005M NaBH₄ | 130 | -255 |
| BH₃ - 2MeTHF (0.94M) | 139 | -75 |
| BH₃ - 2MeTHF (0.88M) with 0.005M NaBH₄ | 140 | -71 |
| BH₃ - 2MeTHF (0.94M) with 0.005M LiBH₄ | 152 | -41 |
| BH₃ - 2MeTHF (1.3M) | 125 | -100 |
| BH₃ - 2MeTHF (1.6M) | 128 | -229 |
| BH₃ - 2EMTHF (0.37M) | 158 | -11 |

| | | |
|---|---|---|
| * MeTHF = 2-methyltetrahydrofuran, EMTHF = 2-(ethoxymethyl)-tetrahydrofuran | | |

The DSC measurements were conducted in a sealed cup with a ramp rate of 4 degrees/min. The decomposition occurring is the ether cleavage of the etheral ring of the complexing agent. For approximately 1M solutions of the new borane-2-methyltetrahydrofuran complex the energy release is less than one third of that for borane-tetrahydrofuran, giving the new compound a significant safety advantage over the standard commercial tetrahydrofuran complex because of the lower decomposition energy released. Furthermore, even less energy is released at a higher onset temperature for the sample of borane-2-methyltetrahydrofuran complex in 2-methyltetrahydrofuran containing a low concentration of lithium borohydride.

An isothermal DSC was run on 12 mg of a 1.3M solution of borane-2-methyltetrahydrofuran complex in 2-methyltetrahydrofuran at 55°C for 3000 minutes to observe occurrence of thermal events. No thermal events were seen over this time period.

The present invention further provides a method of using the new borane ether complexes for organic reactions. The method comprises the step of contacting a borane ether complex and a substrate in a reaction vessel and preventing the escape of evolved gaseous diborane from the reaction vessel. Preferably, the reaction vessel containing the borane ether complex and the substrate is equipped with a back-pressure regulator and maintained at a pressure greater than approximately atmospheric pressure. More preferably, the pressure is in the range of approximately 300 mbar to approximately 7000 mbar higher than atmospheric pressure. Even more preferably, the pressure is in the range of approximately 300 mbar to approximately 2500 mbar higher than atmospheric pressure. The advantages provided by preventing escape of diborane from the reaction vessel include a more efficient use of borane, thereby eliminating the need to use excess borane and less formation of by-products during reaction.

In the presence of a suitable substrate, the new borane ether complexes of the present invention react readily and preferentially with the desired compound. Under these conditions thermal decomposition and ring opening reactions are negligible generating only insignificant amounts of by-products.

Organic reactions, for which the new borane ether complexes can be employed according to the invention, include especially the reduction of functional groups and hydroboration reactions with alkenes and alkynes. Furthermore, suitable substrates to be used in reduction reactions with the new borane ether complexes include organic compounds with aldehyde, ketone, lactone, epoxide, ester, amide, oxime, imine, carboxylic acid and nitrile groups. Advantageously, the new borane ether complexes can be used for enantioselective reductions of prochiral ketones and prochiral imines in the presence of chiral oxazaborolidine catalysts like MeCBS (a methyl-substituted chiral oxazaborolidine named after Corey, Bakshi and Shibata, c.f. Corey, E.J. et al., Angew. Chem. Int. Ed., 37, 1986-2012 (1998)).

Asymmetric reduction using chiral oxazaborolidine catalysts is an excellent tool for the synthesis of secondary alcohols in high enantiomeric excess (Catalysis of Fine Chemical Synthesis, Roberts, S.M.; Poignant, G., (Eds.), Wiley, & Sons, Ltd.: New York 2002.). The enantioselective borane reduction of prochiral ketones catalyzed by chiral oxazaborolidine compounds has effectively competed with enzymatic and transition metal catalyzed hydrogenation reactions, because of the mild reaction conditions, high enantioselectivity, predictability and high yields. The reduction is highly efficient and operationally simple, therefore is well suited to an industrial setting. Several oxazaborolidine compounds have been used in the scale-up of pharmaceutical compounds. Patents cover the synthesis and use of oxazaborolidine catalysts, e. g. US 4,943,635, US 5,189,177, US 5,264,574, US 5,264,585, US 5,552,548, US 6,005,133 and US 6,037,505.

The precise stereocontrol of the reduction arises from a cyclic transition state where the oxazaborolidine holds the ketone via coordination to the Lewis acidic boron while the borane is held in proximity by the amine of the catalyst. Generally 2-10 mole% of oxazaborolide catalyst is used along with a borane source such as borane-tetrahydrofuran, borane-dimethylsufide or borane-diethylaniline complexes. The ketone is usually added slowly to the mixture of catalyst and borane. Simultaneous addition of borane and ketone to the catalyst is also effective for optimizing enantioselectivity. Several factors affecting the enantioselectivity of MeCBS-catalysed ketone reductions with borane-tetrahydrofuran complex are outlined in US 6,218,585. The presence of sodium borohydride as the stabilizer in commercial borane-tetrahydrofuran has been shown to be detrimental to the enantioselectivity of oxazaborolidine catalyzed reductions. Typically when sodium borohydride stabilized borane-tetrahydrofuran complex is used in the MeCBS catalyzed reduction of acetophenone, an enantioselectivity of 85-90% ee is obtained in the reduction. Borohydride is a competitive non-selective catalyst for ketone reductions (Jockel, H.; Schmidt, R., J. Chem. Soc. Perkin Trans. 2 (1997), 2719-2723.), thus deactivation of the sodium borohydride with an acidic compound is essential for high enantioselectivity when using borane-tetrahydrofuran. Contrary to the work of Matos and co-workers disclosed in US 6,218,585, we have found that the presence of lithium borohydride as a stabilizer in borane-2-methyltetrahydrofuran solutions does not have a detrimental affect on the enantioselectivity of the oxazaborolidine catalyzed ketone reduction. (for examples, see the results in Table 2). Of course, analogous results have been obtained with both enantiomers of the catalyst, i.e. (R)-MeCBS and (S)-MeCBS.

The reduction using borane-2-methyltetrahydrofuran containing lithium borohydride is also faster compared to borane-2-methyltetrahydrofuran without borohydride. Examples 9 and 10 contained from 16 - 17 % acetophenone whereas example 11 showed complete reduction. When the reaction using borane-2-methyltetrahydrofuran without lithium borohydride was allowed to stir for additional 20 minutes after the ketone addition, the reduction reached completion and enantioselectivity was excellent, example 13. Decreasing the ketone addition time from 2 hours to 30 minutes also gave an excellent enantioselectivity in the acetophenone reduction, example 14.

Compared to the standard literature procedure using sodium borohydride stabilized borane-tetrahydrofuran as reducing agent, higher enantiomeric excesses are observed in reductions with the new borane ether complexes of the present invention. Even the presence of sodium borohydride did not substantially decrease the enantioselectivity of the ketone reduction with the borane-2-methyltetrahydrofuran complex and MeCBS, example 15.

**Table 2:**

| Example No. | Borane-2-methyltetrahydrofuran | % acetophenone remaining | % ee of phenethanol |
|---|---|---|---|
| 9 | without LiBH₄, prep. acc. to Ex. 5 | 17 | 96.0 |
| 10 | without LiBH₄, prep. acc. to Ex. 7 | 16 | 94.4 |
| 11 | with LiBH₄, prep. acc. to Ex. 5 | 0 | 94.8 |
| 12 (comparative) | Borane - THF without hydride stabili-zation | 0.7 | 95.2 |
| 13 | without LiBH₄, prep. acc. to Ex. 6 | 0 * | 96.8 |
| 14 | without LiBH₄, prep. acc. to Ex. 7 | 0 ** | 93.7 |
| 15 | with NaBH₄, prep. acc. to Ex. 1 | 6.2* | 93.2 |

| | | | |
|---|---|---|---|
| * Sampled 30 minutes after ketone addition ** 30 minute ketone addition time and 2 h hold before quenching | | | |

Prior to this invention, borane-tetrahydrofuran complex for use in oxazaborolidine catalyzed asymmetric reduction of ketones was not commercially available in an unstabilized form. The present invention allows for preparation of stabilized and unstabilized borane solutions as the 2-methyltetrahydrofuran complex that can be used with excellent results for oxazaborolidine catalyzed asymmetric reduction of ketones and imines.

### Examples

The following examples illustrate the present invention without limitation of the same. Borane concentrations were measured by titration of the borane with acid according the method described by Brown, H.C.; Kramer, G.W.; Levy, A.B.; Midland, M.M. in Organic Synthesis via Boranes, John Wiley and Sons, Inc., New York 1973, pp 241-244.

### Example 1: Synthesis of borane complex of 2-methyltetrahydrofuran

A glass reactor was purged with nitrogen and charged with 422.6 g of 2-methyltetrahydrofuran (distilled from potassium). The content of the vessel was cooled to 0°C. The back-pressure regulator of the reactor was set at 4400 mbar. Diborane (8 g) was bubbled into the reactor over a 40 minute period of time. The reactor temperature reached a maximum of 4.5°C and a head pressure of 1400 mbar. Upon completion of the diborane addition, the reactor solution was allowed to stir overnight. The ¹¹B NMR spectrum showed a quartet at δ = -1.2 ppm (95%, ¹J(¹¹B,¹H) = 106 Hz) assigned to the product and a second signal at δ = 18 ppm (5 %, singlet) assigned to a borate impurity. The density of the solution was 0.848 g/ml at 22°C and the borane concentration 0.88 M.

The solution was then divided into two halves. The one half of the solution was stabilized with NaBH₄ (0.05 g). After the addition of the NaBH₄, the solution was stirred for 24 hours in order for the NaBH₄ to dissolve. Both the stabilized and unstabilized halves were then split into two equal portions for stability studies at room temperature and 0-5°C (c.f. Figures 1 - 4).

### Example 2: Synthesis of borane complex of 2-methyltetrahydrofuran

A glass reactor was purged with nitrogen and charged with 430 g of 2-methyltetra-hydro-furan (Aldrich, used as received). The content of the vessel was cooled to 0°C. The back-pressure regulator of the reactor was set at 4400 mbar. Diborane (10 g) was bubbled into the reactor over a 37 minute period of time. The reactor temperature reached a maximum of 4.6°C and a head pressure of 1700 mbar. Sodium borohydride (0.09 g) was added to the solution. The ¹¹B NMR spectrum showed a quartet at δ = -1.0 ppm (95 %, ¹J(¹¹B,¹H) = 106 Hz), borate at δ = 18 ppm (4.5 %, singlet) and a trace of NaB₃H₈ at δ = -26 ppm. The density of the solution was 0.848 g/ml at 22°C. The borane concentration was 0.94M.

### Example 3: Synthesis of borane complex of 2,5-dimethyltetrahydrofuran

Diborane (0.2 g, 14 mmol of BH₃) was added to a sample of 2,5-dimethyltetrahydrofuran (4.9 g, 5.9 ml) in a flask in an ice bath. The ¹¹B NMR spectrum of the mixture clearly showed the borane complex of 2,5-dimethyltetrahydrofuran (62%) at δ = -1.5 ppm (q, ¹J(¹¹B,¹H) = 104 Hz) and also dissolved diborane (24%) as a multiplet at 6 = 17.9 ppm (¹J(¹¹B,¹H) = 120, 60 Hz). The amount of dialkoxyborane initially formed was about 14% (δ = 28 ppm, d, ¹J(¹¹B,¹H) = 104 Hz). The excess diborane was not purged and the sample was kept at 0°C. Monitoring the sample over 6 days at 0°C showed relatively little change with the complexed borane maintaining at about 60% by ¹¹B NMR. The sample was then left at ambient temperature to monitor ether ring-opening, see Figure 6.

### Example 4: Synthesis of borane complex of 2-(ethoxymethyl)-tetrahydrofuran

Diborane (1.3 g, 94 mmol BH₃) was added to 100 ml of 2-(ethoxymethyl)-tetrahydrofuran at 0°C. The ¹¹B NMR spectrum of the mixture clearly showed the borane complex of 2-(ethoxymethyl)-tetrahydrofuran at δ = -0.96 ppm (broad q, ¹J(¹¹B,¹H) = 96 Hz, 71%) but also dissolved diborane as a multiplet at δ = 17.9 ppm (¹J(¹¹B,¹H) = 120, 60 Hz, 26%) and a third signal at δ = 29 ppm (d, ¹J(¹¹B,¹H) = 177 Hz, 3%) assigned to dialkoxyborane. Based on the spectral integration and the amount of diborane added, the concentration of borane complex of 2-(ethoxymethyl)-tetrahydrofuran is 0.66M. The concentration of dissolved diborane is about 0.12M. Additional 2-(ethoxymethyl)-tetrahydrofuran (100 ml) was added to complex the dissolved diborane. The ¹¹B NMR spectrum of the mixture now showed 7-9.6% of borane-2-(ethoxymethyl)-tetrahydrofuran complex, 6.1% dialkoxyborane and only 14% dissolved diborane. Therefore the concentration of borane-2-(ethoxymethyl)-tetrahydrofuran complex was approximately 0.37 M.

### Example 5: Synthesis of borane complex of 2-methyltetrahydrofuran

A reactor was purged with nitrogen and charged with 423 g of 2-methyltetrahydrofuran (Penn Specialty Lot #2-5613). The content of the vessel was cooled to -12 °C. The back-pressure regulator of the reactor was set at 4400 mbar. Diborane (16 g) was added to the reactor over a 95 minute period of time. The reactor temperature reached a maximum of 8.9°C and a head pressure of 2000 mbar. Upon completion of the diborane addition, it was determined that excess diborane had been added; borane titration showed 1.48M. The reactor solution diluted with additional 2-methyltetrahydrofuran (250 ml) to bring the concentration down to 1 M and allowed to stir overnight. The ¹¹B NMR spectrum indicated a borate concentration of 2.1%. The solution was then divided into two halves. The one half was stabilized with LiBH₄ (0.037g). The LiBH₄ slowly dissolved, and a small peak was seen at δ = -29 ppm for B₃H₈-Li⁺ in the ¹¹B NMR spectrum. Both the stabilized and unstabilized halves were then split into two equal portions for stability studies at room temperature and 0°C in cylinders with gauges and dip tubes. The ¹¹B NMR spectrum showed a quartet at δ = -1.5 ppm (¹J(¹¹B,¹H) = 106 Hz). The density of the clear colorless solution was 0.842 g/ml at 22°C. The concentration was 0.96M

### Example 6: Synthesis of borane complex of 2-methyltetrahydrofuran

A glass reactor was purged with nitrogen and charged with 430 g of 2-methyltetra-hydro-furan (Penn Specialty Lot #2-5613). The contents of the vessel were cooled to - 3°C. The back-pressure regulator of the reactor was set at 4400 mbar. Diborane (10 g) was bubbled into the reactor over a 60 minute period of time. The reactor temperature reached a maximum of -0.8°C and a head pressure of 1800 mbar. Upon completion of the diborane addition, it was determined by ¹¹B NMR that 5.5% of a borate impurity was present.

### Example 7: Synthesis of borane complex of 2-methyltetrahydrofuran

A glass reactor was purged with nitrogen and charged with 423 g of 2-methyltetra-hydro-furan (Penn Specialty Lot #2-5613). The contents of the vessel were cooled to-3°C. The back-pressure regulator of the reactor was set at 4400 mbar. Diborane (10 g) was fed to the headspace of the reactor over a 60 minute period of time. The reactor temperature reached a maximum of -0.5°C and a head pressure of 2000 mbar. Upon completion of the diborane addition, it was determined by ¹¹B NMR that 5% of a borate impurity was present. The density was measured at 0.844 g/ml. The borane concentration was 1.3M.

### Example 8: Hydroboration of 1-octene with borane complex of 2-methyltetrahydrofuran

Reaction of borane complex of 2-methyltetrahydrofuran (4 mmol, unstabilized) with 1-octene (1.3 g, 11.6 mmol, 1:3 mole ratio of BH₃ to alkene) in toluene at ambient temperature showed 100% conversion of borane - 2-methyltetrahydrofuran to products after 30 minutes. The reaction was exothermic. Trialkylborane (99%) was seen at δ = 89 ppm in the ¹¹B NMR spectrum.

### Examples 9 - 12: Asymmetric reductions of acetophenone

The following method was used for the asymmetric reductions of acetophenone, results are shown in Table 2. Acetophenone was added by syringe pump (2 ml in 17 ml of THF, i.e. 17 mmol) over 2 hours to a solution of 10 mmol of the respective borane complex (e.g. 10 ml of a 1 M solution) and 5 mol% (relative to the acetophenone) (R)-MeCBS in toluene at room temperature. After stirring for 10 min. following the ketone addition, HCl (1 M, 10 ml) was added to quench the reaction. The phenethanol and any unreacted acetophenone were extracted with 20 ml anhydrous diethyl ether. The organic layer was washed with saturated KCl and saturated NaHCO₃ solutions, then dried over Na₂SO₄. Chiral GC analysis showed area % acetophenone (if remaining) and ratio of (R)-phenethanol to (S)-phenethanol, reported as %ee in Table 2.

### Examples 13 and 15: Asymmetric reductions of acetophenone with longer hold time

Acetophenone was added by syringe pump (2 ml in 17 ml of THF, i.e. 17 mmol) over 2 hours to a solution of 10 mmol of the respective borane complex (e.g. 11.4 ml of a 0.88M solution) and 5 mol% (relative to the acetophenone) (R)-MeCBS in toluene at room temperature. After stirring for 30 min. following the ketone addition, HCl (1 M, 10 ml) was added to quench the reaction. The phenethanol and any unreacted acetophenone were extracted with 20 ml anhydrous diethyl ether. The organic layer was washed with saturated KCl and saturated NaHCO₃ solutions, then dried over Na₂SO₄. Chiral GC analysis showed area % acetophenone (if remaining) and ratio of (R)-phenethanol to (S)-phenethanol (see Table 2).

### Example 14: Asymmetric reductions of acetophenone (fast addition) with longer hold time

Acetophenone was added by syringe pump (2 ml in 17 ml of THF, i.e. 17 mmol) over 30 minutes to a solution of 10 mmol of the borane complex (7.7 ml of a 1.3M solution) and 5 mol% (relative to the acetophenone) (R)-MeCBS in toluene at room temperature. After stirring for 2 hours following the ketone addition, HCl (1 M, 10 ml) was added to quench the reaction. The phenethanol and any unreacted acetophenone were extracted with 20 ml anhydrous diethyl ether. The organic layer was washed with saturated KCl and saturated NaHCO₃ solutions, then dried over Na₂SO₄. Chiral GC analysis showed area % acetophenone (if remaining) and ratio of (R)-phenethanol to (S)-phenethanol (see Table 2).

### Example 16: Reduction of benzoic acid with borane complex of 2-methyltetrahydrofuran

12.21 g (0.1 mol) of benzoic acid in 40 ml 2-methyltetrahydrofuran were added over 1 hour via a syringe to 125 ml of a 1 M solution of BH₃ - 2-MeTHF (0.125 mol) at 0°C. After the addition was complete and the hydrogen evolution had ceased, the mixture was warmed to room temperature. After stirring for 2 hours 2 ml of water were added and the mixture was extracted with 100 ml of saturated aqueous Na₂CO₃ solution. Analysis of the organic layer gave a yield of 97.1 % of benzyl alcohol and a water content of 5.3%.

### Example 17 (comparative): Reduction of benzoic acid with borane complex of tetrahyd rofu ra n

12.21 g (0.1 mol) of benzoic acid in 20 ml tetrahydrofuran were added over 1 hour via a syringe to 125 ml of a 1M solution of BH₃- THF (0.125 mol) at 0°C. After the addition was complete and the hydrogen evolution had ceased, the mixture was warmed to room temperature. After stirring for 2 hours 2 ml of water were added and the mixture was extracted with 100 ml of saturated aqueous Na₂CO₃ solution. Analysis of the organic layer gave a yield of 62.0 % of benzyl alcohol and a water content of 11.0%.

### Example 18: Reduction of propanoic acid with borane complex of 2-methyltetrahydrofuran

7.41 g (0.1 mol) of propanoic acid in 15 ml 2-methyltetrahydrofuran were added over 1 hour via a syringe to 125 ml of a 1M solution of BH₃ - 2-MeTHF (0.125 mol) at 0°C. After the addition was complete and the hydrogen evolution had ceased, the mixture was warmed to room temperature. After stirring for 1 hour 2 ml of water were added and the mixture was extracted with 100 ml of saturated aqueous Na₂CO₃ solution. The aqueous layer was extracted with 100 ml of 2-methyltetrahydrofuran. Analysis of the combined organic layers gave a yield of 96.5 % of n-propanol and a water content of 5.0%.

### Example 19 (comparative): Reduction of propanoic acid with borane complex of tetrahydrofuran

7.41 g (0.1 mol) of propanoic acid in 15 ml tetrahydrofuran were added over 1 hour via a syringe to 125 ml of a 1 M solution of BH₃ - THF (0.125 mol) at 0°C. After the addition was complete and the hydrogen evolution had ceased, the mixture was warmed to room temperature. After stirring for 1 hour 2 ml of water were added and the mixture was extracted with 100 ml of saturated aqueous Na₂CO₃ solution. The aqueous layer was extracted with 100 ml of 2-methyltetrahydrofuran. Analysis of the combined organic layers gave a yield of 73.6 % of n-propanol and a water content of 20.4 %.

### Example 20: Reduction of heptanenitrile with borane complex of 2-methyltetrahydrofuran

0.02 mol of heptanenitrile in 10 ml 2-methyltetrahydrofuran were added over 1 hour via a syringe to 25 ml of a 1 M solution of BH₃ - 2-MeTHF (0.025 mol) at 0°C. After the addition was complete the mixture was heated to reflux for 3 hours. After cooling to 0°C again 0.8 ml of methanol were slowly added, and after the hydrogen evolution had ceased 20 ml of 1 M hydrogen chloride were added and the mixture was refluxed again. After 30 minutes the mixture was cooled to room temperature and extracted with 40 ml of saturated aqueous Na₂CO₃ solution. The aqueous layer was extracted with 40 ml of diethyl ether. Analysis of the combined organic layers gave a yield of 84.5 % of heptylamine and a water content of 5.9%.

### Example 21 (comparative): Reduction of heptanenitrile with borane complex of tetrahydrofuran

0.02 mol of heptanenitrile in 10 ml tetrahydrofuran were added over 1 hour via a syringe to 25 ml of a 1 M solution of BH₃ - THF (0.025 mol) at 0°C. After the addition was complete the mixture was heated to reflux for 3 hours. After cooling to 0°C again 0.8 ml of methanol were slowly added, and after the hydrogen evolution had ceased 20 ml of 1 M hydrogen chloride were added and the mixture was refluxed again. After 30 minutes the mixture was cooled to room temperature and extracted with 40 ml of saturated aqueous Na₂CO₃ solution. The aqueous layer was extracted with 40 ml of diethyl ether. Analysis of the combined organic layers gave a yield of 80.7 % of heptylamine and a water content of 30.0 %.

### Example 22: Reduction of N,N-dimethylbenzamide with borane complex of 2-methyltetrahydrofuran

98 ml of a 1 M solution of BH₃ - 2-MeTHF (0.098 mol) were added over 1 minute to 11.48 g (0.085 mol) of N,N-dimethylbenzamide in 100 ml 2-methyltetrahydrofuran at ambient temperature and stirred for 12 hours. After cooling to 0°C 8 ml of methanol were slowly added and then the mixture was extracted with 100 ml of saturated aqueous Na₂CO₃ solution. The organic layer was dried over sodium sulfate. Analysis of the organic layer gave a yield of 96.3 % of dimethylbenzylamine.

### Example 23 (comparative): Reduction of N,N-dimethylbenzamide with borane complex of tetrahydrofuran

98 ml of a 1 M solution of BH₃ - THF (0.098 mol) were added over 1 minute to 11.48 g (0.085 mol) of N,N-dimethylbenzamide in 100 ml tetrahydrofuran at ambient temperature and stirred for 12 hours. After cooling to 0°C 8 ml of methanol were slowly added and then the mixture was extracted with 100 ml of saturated aqueous Na₂CO₃ solution. The organic layer was dried over sodium sulfate. Analysis of the organic layer gave a yield of 95.1 % of dimethylbenzylamine.

## Claims

1. Borane ether complexes of the formula 1, 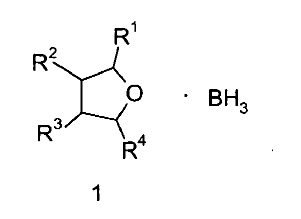 wherein
R¹ to R⁴ represent independently from each other hydrogen, C₁ - C₄-alkyl, C₃ - C₆-cycloalkyl or a substituent of the formula CH₂OR⁵ , wherein R⁵ is C₁ - C₄-alkyl or C₃ - C₆-cycloalkyl,
or two adjacent substituents R¹ to R⁴ together are a divalent group selected from the group consisting of -CH₂CH₂-, -CH(CH₃)CH₂-, -CH₂CH₂CH₂-, -CH(CH₃)CH(CH₃)-, -CH(CH₂CH₃)CH₂-, -C(CH₃)₂C(CH₃)₂-, -CH₂C(CH₃)₂CH₂- and -(CH₂)₆- to form with the -CH-CH- moiety of the tetrahydrofuran ring a cyclic structure,
with the provision that at least one of the substituents R¹ to R⁴ is not hydrogen.

2. Borane ether complex according to claim 1, wherein R¹ is methyl and R² to R⁴ each are hydrogen.

3. Solutions comprising at least one borane ether complex according to claim 1 and at least one solvent.

4. Solutions according to claim 3 where the solvent comprises the ether used to complex the borane in the borane ether complex with the chemical structure 1.

5. Solutions according to claim 3 or 4 with a concentration of the borane ether complex in the range of 0.01 to 3 mol/l.

6. A method of using borane ether complexes according to claim 1 for organic reactions.

7. A method of using borane ether complexes according to claim 6, wherein the organic reaction is a reduction or a hydroboration reaction.

8. A method of using borane ether complexes according to claim 1 comprising the step of contacting a borane ether complex and a substrate in a reaction vessel and preventing the escape of evolved gaseous diborane from the reaction vessel.

9. A method of using borane ether complexes according to claim 1 for asymmetric reductions of prochiral ketones, imines or oximes comprising additionally using a chiral catalyst.

10. A method of using borane ether complexes according to claim 1 for asymmetric reductions of prochiral ketones, imines or oximes comprising additionally using a chiral oxazaborolidine catalyst.

11. A method of using borane ether complexes according to claim 9 or 10 comprising addition of at least one alkali metal borohydride as a stabilizing agent.

12. A method of using borane ether complexes according to claim 11 comprising addition of lithium borohydride as a stabilizing agent.

13. A method of using borane ether complexes according to claim 11 comprising addition of sodium borohydride as a stabilizing agent.

## Patentansprüche

1. Boran-Ether-Komplexe der Formel 1, 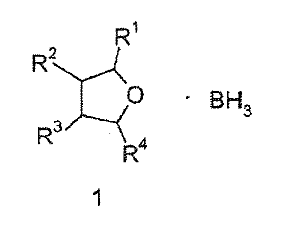 worin
R¹ bis R⁴ unabhängig voneinander für Wasserstoff, C₁-C₄-Alkyl, C₃-C₆-Cycloalkyl oder einen Substituenten der Formel CH₂OR⁵, worin R⁵ für C₁-C₄-Alkyl oder C₃-C₆-Cycloalkyl steht, stehen,
oder zwei benachbarte Substituenten R¹ bis R⁴ zusammen für eine zweiwertige Gruppe aus der Gruppe bestehend aus -CH₂CH₂-, -CH(CH₃)CH₂-, -CH₂CH₂CH₂-, -CH(CH₃)CH(CH₃)-, -CH (CH₂CH₃)CH₂-, -C(CH₃)₂C(CH₃)₂-, -CH₂C(CH₃)₂CH₂- und -(CH₂)₆- stehen und zusammen mit der -CH-CH-Gruppierung des Tetrahydrofuranrings eine cyclische Struktur bilden;
mit der Maßgabe, daß mindestens einer der Substituenten R¹ bis R⁴ nicht für Wasserstoff steht.

2. Boran-Ether-Komplex nach Anspruch 1, worin R¹ für Methyl steht und R² bis R⁴ jeweils für Wasserstoff stehen.

3. Lösungen, die mindestens einen Boran-Ether-Komplex nach Anspruch 1 und mindestens ein Lösungsmittel umfassen.

4. Lösungen nach Anspruch 3, wobei das Lösungsmittel den zur Komplexierung des Borans in dem Boran-Ether-Komplex mit der chemischen Struktur 1 verwendeten Ether umfaßt.

5. Lösungen nach Anspruch 3 oder 4 mit einer Konzentration des Boran-Ether-Komplexes im Bereich von 0,01 bis 3 mol/l.

6. Verfahren zur Verwendung von Boran-Ether-Komplexen nach Anspruch 1 für organische Reaktionen.

7. Verfahren zur Verwendung von Boran-Ether-Komplexen nach Anspruch 6, bei dem es sich bei der organischen Reaktion um eine Reduktions- oder Hydroborierungsreaktion handelt.

8. Verfahren zur Verwendung von Boran-Ether-Komplexen nach Anspruch 1, bei dem man einen Boran-Ether-Komplex und ein Substrat in einem Rekationsgefäß in Berührung bringt und das Entweichen von entwickeltem gasförmigem Diboran aus dem Reaktionsgefäß verhindert.

9. Verfahren zur Verwendung von Boran-Ether-Komplexen nach Anspruch 1 für asymmetrische Reduktionen von prochiralen Ketonen, Iminen oder Oximen, bei dem man zusätzlich einen chiralen Katalysator verwendet.

10. Verfahren zur Verwendung von Boran-Ether-Komplexen nach Anspruch 1 für asymmetrische Reduktionen von prochiralen Ketonen, Iminen oder Oximen, bei dem man zusätzlich einen chiralen Oxazaborolidin-Katalysator verwendet.

11. Verfahren zur Verwendung von Boran-Ether-Komplexen nach Anspruch 9 oder 10, bei dem man mindestens ein Alkalimetallborhydrid als Stabilisierungsmittel zusetzt.

12. Verfahren zur Verwendung von Boran-Ether-Komplexen nach Anspruch 11, bei dem man als Stabilisierungsmittel Lithiumborhydrid zusetzt.

13. Verfahren zur Verwendung von Boran-Ether-Komplexen nach Anspruch 11, bei dem man als Stabilisierungsmittel Natriumborhydrid zusetzt.

## Revendications

1. Complexes borane-éther de formule 1, 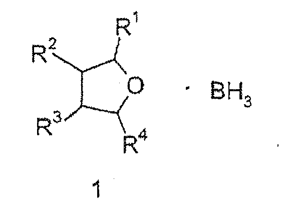 dans laquelle
R¹ à R⁴ représentent indépendamment l'un de l'autre un atome d'hydrogène ou un groupe alkyle en C₁-C₄, cycloalkyle en C₃-C₆ ou un substituant de formule CH₂OR⁵, dans laquelle R⁵ représente un groupe alkyle en C₁-C₄ ou cycloalkyle en C₃-C₆,
ou deux substituants R¹ à R⁴ adjacents forment ensemble un groupe divalent choisi dans le groupe constitué par -CH₂CH₂- , -CH(CH₃)CH₂-, -CH₂CH₂CH₂-, -CH(CH₃)CH(CH₃)-, -CH (CH₂CH₃)CH₂-, -C(CH₃)₂C(CH₃)₂-, -CH₂C(CH₃)₂CH₂- et -(CH₂)₆- pour former, avec le groupement -CH-CH- du noyau tétrahydrofurane, une structure cyclique,
sous réserve qu'au moins un des substituants R¹ à R⁴ ne représente pas un atome d'hydrogène.

2. Complexe borane-éther selon la revendication 1, dans lequel R¹ représente un groupe méthyle et R² à R⁴ représentent chacun des atomes d'hydrogène.

3. Solutions comprenant au moins un complexe borane-éther selon la revendication 1 et au moins un solvant.

4. Solutions selon la revendication 3, dans lesquelles le solvant comprend l'éther utilisé pour complexer le borane dans le complexe borane-éther ayant la structure chimique 1.

5. Solutions selon la revendication 3 ou 4, ayant une concentration du complexe borane-éther sur la gamme de 0,01 à 3 mol/l.

6. Procédé d'utilisation de complexes borane-éther selon la revendication 1 pour des réactions organiques.

7. Procédé d'utilisation de complexes borane-éther selon la revendication 6, dans lequel la réaction organique est une réaction de réduction ou d'hydroboration.

8. Procédé d'utilisation de complexes borane-éther selon la revendication 1, comprenant l'étape consistant à mettre en contact un complexe borane-éther et un substrat dans un réacteur et à empêcher que le diborane gazeux dégagé ne s'échappe du réacteur.

9. Procédé d'utilisation de complexes borane-éther selon la revendication 1 pour des réductions asymétriques de cétones, d'imines ou d'oximes prochirales, comprenant en outre le fait d'utiliser un catalyseur chiral.

10. Procédé d'utilisation de complexes borane-éther selon la revendication 1 pour des réductions asymétriques de cétones, d'imines ou d'oximes prochirales, comprenant en outre le fait d'utiliser un catalyseur oxazaborolidine chiral.

11. Procédé d'utilisation de complexes borane-éther selon la revendication 9 ou 10, comprenant le fait d'ajouter au moins un borohydrure de métal alcalin en tant qu'agent stabilisant.

12. Procédé d'utilisation de complexes borane-éther selon la revendication 11, comprenant le fait d'ajouter du borohydrure de lithium en tant qu'agent stabilisant.

13. Procédé d'utilisation de complexes borane-éther selon la revendication 11 comprenant le fait d'ajouter du borohydrure de sodium en tant qu'agent stabilisant.
